# EUROPÄISCHE PATENTSCHRIFT

(11) **EP 3 308 163 B1**
(45) Veröffentlichungstag und Bekanntmachung des Hinweises auf die Patenterteilung: **05.03.2025**
(21) Anmeldenummer: 16729850.4
(22) Anmeldetag: 09.06.2016
(51) Int. Cl.: G01N 33/50, G01N 33/574, G01N 33/68

(54) **VERFAHREN UND VORRICHTUNG ZUR ÜBERWACHUNG DES GESUNDHEITSZUSTANDS VON MILCHKÜHEN**
METHOD AND APPARATUS FOR MONITORING THE STATE OF HEALTH OF DAIRY COWS
PROCÉDÉ ET DISPOSITIF POUR SURVEILLER L'ÉTAT DE SANTÉ DE VACHES LAITIÈRES

(30) Priorität: 10.06.2015 DE 102015007366
(43) Veröffentlichungstag der Anmeldung: 18.04.2018
(73) Patentinhaber: Fraunhofer-Gesellschaft zur Förderung der angewandten Forschung e.V., 80686 München (DE)
(72) Erfinder: LEHMANN, Jörg, 04451 Borsdorf (DE); ZOLDAN, Katharina, 79108 Freiburg (DE)
(74) Vertreter: Kuttenkeuler, David
(86) Internationale Anmeldenummer: PCT/EP2016/063109
(87) Internationale Veröffentlichungsnummer: WO 2016/198501

(56) Entgegenhaltungen:
- WO-A1-01/27631
- DATABASE BIOSIS [online] BIOSCIENCES INFORMATION SERVICE, PHILADELPHIA, PA, US; June 2013 (2013-06-01), KALMUS P ET AL: "Milk haptoglobin, milk amyloid A, and N-acetyl-beta-D-glucosaminidase activity in bovines with naturally occurring clinical mastitis diagnosed with a quantitative PCR test", Database accession no. PREV201300452970
- JOURNAL OF DAIRY SCIENCE, vol. 96, no. 6, June 2013 (2013-06-01), pages 3662 - 3670, ISSN: 0022-0302(print), DOI: 10.3168/JDS.2012-6177
- GROOT DE N ET AL: "Over-expression of the murine polymeric immunoglobulin receptor gene in the mammary gland of transgenic mice", TRANSGENIC RESEARCH, SPRINGER NETHERLANDS, NL, vol. 8, no. 2, 1 April 1999 (1999-04-01), pages 125 - 135, XP002227113, ISSN: 0962-8819, DOI: 10.1023/A:1008981312682
- KATHARINA ZOLDAN ET AL: "Increase of CD25 expression on bovine neutrophils correlates with disease severity in post-partum and early lactating dairy cows", DEVELOPMENTAL AND COMPARATIVE IMMUNOLOGY, vol. 47, no. 2, 1 December 2014 (2014-12-01), US, pages 254 - 263, XP055287384, ISSN: 0145-305X, DOI: 10.1016/j.dci.2014.08.002
- MOHAMED EZZAT ALNAKIP ET AL: "The Immunology of Mammary Gland of Dairy Ruminants between Healthy and Inflammatory Conditions", JOURNAL OF VETERINARY MEDICINE, vol. 56, no. 3, 1 January 2014 (2014-01-01), pages 313 - 31, XP055287422, ISSN: 2356-7708, DOI: 10.1155/2014/659801
- ULRIKA GRÖNLUND ET AL: "Haptoglobin and serum amyloid A in milk from dairy cows with chronic sub-clinical mastitis", VETERINARY RESEARCH., vol. 36, no. 2, 1 March 2005 (2005-03-01), NL, pages 191 - 198, XP055287379, ISSN: 0928-4249, DOI: 10.1051/vetres:2004063
- KALMUS P ET AL: "Milk haptoglobin, milk amyloid A, and N-acetyl-beta-D-glucosaminidase activity in bovines with naturally occurring clinical mastitis diagnosed with a quantitative PCR test", JOURNAL OF DAIRY SCIENCE, vol. 96, no. 6, June 2013 (2013-06-01), pages 3662 - 3670, XP002759760
- BOEHMER J L ET AL: "The proteomic advantage: Label-free quantification of proteins expressed in bovine milk during experimentally induced coliform mastitis", VETERINARY IMMUNOLOGY AND IMMUNOPATHOLOGY, ELSEVIER BV, AMSTERDAM, NL, vol. 138, no. 4, 15 December 2010 (2010-12-15), pages 252 - 266, XP027552563, ISSN: 0165-2427, [retrieved on 20101014], DOI: 10.1016/J.VETIMM.2010.10.004
- ROZAIHAN MANSOR: "Glasgow Theses Service", 1 February 2012 (2012-02-01), University of Glasgow, pages 1 - 335, XP055287682, Retrieved from the Internet <URL:http://theses.gla.ac.uk/3207/1/2012MansorPhD.pdf> [retrieved on 20160712]
- SUSUMU MAKIMURA ET AL: "Quantitative determination of bovine serum haptoglobin and its elevation in some inflammatory diseases.", NIHON-JUIGAKU-ZASSHI = JAPANESE JOURNAL OF VETERINARY SCIENCE, vol. 44, no. 1, 1 February 1982 (1982-02-01), JP, pages 15 - 21, XP055287540, ISSN: 0021-5295, DOI: 10.1292/jvms1939.44.15
- ASHOUR M-B A ET AL: "Use of a 96-well microplate reader for measuring routine enzyme activities", ANALYTICAL BIOCHEMISTRY, ACADEMIC PRESS INC, NEW YORK, vol. 166, no. 2, 1 November 1987 (1987-11-01), pages 353 - 360, XP024823103, ISSN: 0003-2697, [retrieved on 19871101], DOI: 10.1016/0003-2697(87)90585-9

## Beschreibung

Die vorliegende Erfindung betrifft Verfahren und Vorrichtungen zur Überwachung des Gesundheitszustands von Milchkühen, insbesondere von ganzen Milchkuhbeständen gemäß der Ansprüche 1 bis 12. Das Verfahren basiert auf der Analyse der Biomarker Haptoglobin (HP) und einem Teil des polymeren Immunglobulinrezeptors (PIGR), der sekretorischen Komponente (*Secretory Component,* SC), in einer Milchprobe. Insbesondere ermöglicht das beanspruchte Verfahren und die Vorrichtung der Erfindung auf Grundlage der hier beschriebenen Protein Biomarker HP und PIGR (SC) eine Diagnose systemischer Erkrankungen, die außerhalb des Euters auftreten. Somit ermöglicht die Erfindung den generellen Gesundheitszustand eines Milchkuhbestands regelmäßig zu überwachen. Die vorliegende Erfindung betrifft nicht-invasive diagnostische Verfahren, sowie Vorrichtungen und diagnostische Kits zur Durchführung dieser Verfahren.

### BESCHREIBUNG

Mit den neuesten technischen Methoden werden immer größere Milchviehbestände kosteneffizient bewirtschaftet. Zudem kann der personelle Aufwand beispielsweise durch den Einsatz von automatischen Melksystemen drastisch reduziert werden. Die tägliche Gesundheitskontrolle der Kühe kann dadurch jedoch nur eingeschränkt oder gar nicht mehr stattfinden. Eine Lösung dieses Problems wäre die Automatisierung der Gesundheitsüberwachung. Das kann über den Nachweis von bestimmten Gesundheitsmarkern in der Milch erfolgen. Als solche Marker bieten sich Akut-Phase-Proteine wie HP an, da deren Konzentrationen schon in der frühen Phase einer Immunantwort sehr schnell ansteigen. Die Messung von HP in Milch wird bisher weder in der Landwirtschaft noch in der veterinärmedizinischen Labordiagnostik routinemäßig eingesetzt.

Die Gesundheitsüberwachung nach der Kalbung wird über eine klinische Untersuchung 7-10 Tage nach der Kalbung sowie die Puerperalkontrolle (zwischen Tag 20 und 28 nach der Kalbung) durch den Herdenmanager oder geschultes Stallpersonal realisiert. Es werden Allgemeinbefinden, Körpertemperatur, Lochien, Milchproduktion und ggf. Milch- oder Harnketone beurteilt. Die Kontrolle der Eutergesundheit erfolgt täglich im Melkstand durch das Melkpersonal. Bei der monatlichen Milchleistungsprüfung werden Parameter wie Zellzahl, Harnstoffgehalt, Fett- und Eiweißgehalt der Milch zum Einschätzen der Eutergesundheit und des Stoffwechselzustandes des individuellen Tiers und der Herde erhoben. Immer mehr Betriebe können am automatischen Melksystem bei jedem Melkvorgang die Zellzahl erfassen. Es gibt Ansätze für die routinemäßige Erfassung von Gesundheitsparametern mittels verschiedener Methoden. Diese können jedoch nur eine Aussage über den Stoffwechselzustand, eine spezielle Erkrankung oder die Eutergesundheit des Tieres liefern.

Die betriebliche Gesundheitsüberwachung stellt sich somit sehr subjektiv und aufwändig dar. Lösungsansätze bieten bisher Aufnahme und Auswertungen von Parameterkombinationen (Leitfähigkeit der Milch, Milchleistung, Bewegungsmuster, Liegezeiten, Progesteronkonzentration, Ketokörper, Laktatdehydrogenase, Fett, Eiweiß, Laktose und Harnstoff in der Milch) mittels entsprechender Messtechnik und multifunktionaler Herdenmanagementprogramme, wie z.B. FullExpert (Lemmer-Fullwood). Das ermöglicht die Detektion von auffälligen Tieren (Brunst, Lahmheit, Fehlgeburt, Labmagenverlagerung (LMV), Ketose, Mastitis). Diese Systeme sind jedoch aufwändig und preisintensiv in der Anschaffung. Bei kritischen Befunden wird ein Tierarzt hinzugezogen. Die Messung klinisch-chemischer, metabolischer und endokrinologischer Parameter im Blut der Tiere gehört in der veterinärmedizinischen Labordiagnostik zur Routineuntersuchung, kann jedoch nur an ausgewählten, auffälligen oder bereits erkrankten Tieren durch den Tierarzt durchgeführt werden. Routinemäßig genutzte Parameter in der klinischen Chemie können nur in Kombination eine Gesamtaussage über den Zustand des Rindes geben.

Es existieren bereits Ansätze, den Gesundheitszustand eines Milchrindes einfacher, schneller und objektiver zu beurteilen. Das erfolgt über die Messung von Akut-Phase-Proteinen im Blut oder in der Milch. HP ist das meistuntersuchte Akut-Phase-Protein im Rind. Während einer Mastitis ist auch in der Milch die HP-Konzentration stark erhöht. HP in der Milch wurde aber bisher nur als potenzieller Indikator für die Mastitis diskutiert.

SC ist kein Akut-Phase-Protein, sondern ein Teil eines Transmembranrezeptors für polymere Immunglobuline, dem PIGR, auf sekretorischen Epithelzellen der Schleimhäute und auch im Euter. Bei Bindung von polymerem Immunglobulin (Ig)A oder IgM wird der Antikörper-Rezeptor-Komplex über Transzytose von der lateralen zur apikalen Seite des Epithels geschleust. Dort wird der Rezeptor enzymatisch gespalten, sodass SC und IgA oder IgM frei werden. Auf diese Weise wird IgA in die Milch transportiert. Während der peripartalen Immunsuppression und in der frühen Laktation sind Milchkühe auch besonders anfällig für Infektionserkrankungen, die nicht am Euter auftreten (systemische Erkrankungen) wie Uterus-, Klauen- oder Atemwegsinfektionen. Nicht selten tritt auch eine LMV auf. Diagnostische Untersuchungen bei diesen Erkrankungen werden routinemäßig bisher im Blut der Tiere durchgeführt. Dazu muss das Tier tierärztlich begutachtet und behandelt werden. Da eine Milchprobe mit wesentlich geringerem Aufwand genommen werden kann, ist es von wirtschaftlichem Interesse für den Landwirt, systemische Erkrankungen in diesem Probenmedium nachweisen zu können.

Grönlund et al. (2005) offenbart das Potential für Haptoglobin und Serum Amyloid A (SAA) in Milch als Indikatoren für chronische subklinische Mastitis und beobachteten erhebliche Schwankung der Haptoglobin- und SAA-Konzentrationen in der Milch in Eutervierteln mit chronischer subklinischer Mastitis.

Kalmus et al. (2013) zeigt den Zusammenhang zwischen quantitativen bakteriologischen Ergebnissen aus einem Echtzeit-PCR-Test und den Konzentrationen von Akute-Phase-Proteinen (APP) und der N-Acetyl-β-d-glucosaminidase (NAGase)-Aktivität in Milch bei natürlich vorkommender klinischer Mastitis.

Boehmer et al. (2010) offenbart proteomischen Ansätze zur Durchführung vergleichender Analysen von Milch von gesunden Kühen und Kühen mit klinischer Mastitis sowie von Proteinen, die in mastitischer Milch identifiziert wurden.

WO 01/27631 A1 betrifft einen Assay zum Nachweis von Haptoglobin (Hp) in Milch als Mittel zur Vorhersage oder zum Nachweis von Mastitis oder subklinischer Mastitis z.B. bei Milchvieh.

Mansor (2012) zeigt Veränderungen von Milchproteomen, Peptidomen und Metabolomen während des Verlaufs der bovinen Mastitis im Vergleich zu normalen Milchproben und untersuchte neue Biomarker für bovine Mastitis.

Makimura et al. (1982) offenbart die Messung von Haptoglobin in Serum zum Nachweis von schweren entzündlichen Erkrankungen wie Mastitis, Poymetra, oder traumatischer Reticulitis, wofür ein modifziertes Verfahren von Tarukoski eingesetzt wurde.

Ashour et al. (1987) offenbart ein Verfahren für die routinemäßige Bestimmung der Geschwindigkeit kolorimetrischer Enzymreaktionen unter Verwendung eines 96-Well-Mikrotiterplatten-Lesegeräts.

Das Gesundheitsmanagement wachsender Milchbetriebe sucht stetig nach alternativen Lösungen zur Überwachung der Herde. Es existieren in der Praxis mehrere Parameter, die es bei täglicher, automatischer Messung im Betrieb ermöglichen, auffällige Kühe zu detektieren (Brunst, Lahmheit, Fehlgeburt, LMV, Ketose, Mastitis). Für die Milch existiert bisher kein validierter Biomarker zur Analyse des allgemeinen Gesundheitszustandes. Die Analyse in der Milch vereinfacht erheblich die Probenahme, dadurch wird eine Messung im Betrieb ermöglicht. Besonders herauszustellen ist, dass hierfür im Gegensatz zur Blutentnahme kein Tierarzt erforderlich ist, was sich positiv auf die Kosten auswirkt und den Gesamtablauf im landwirtschaftlichen Produktionsprozess weniger behindert. Demnach ist es die Aufgabe der vorliegenden Erfindung neue Ansätze zur Gesundheitsüberwachung von Milchkuhbeständen bereitzustellen, die eine vereinfachte und routinemäßige Kontrolle großer Milchrindherden ohne Hinzuziehen eines Veterinärmediziners ermöglicht.

In einem ersten Aspekt wird die gestellte Aufgabe durch ein nicht-invasives Verfahren zur Überwachung des Gesundheitszustandes einer Milchkuh gelöst, wobei das Verfahren nicht zur Diagnose einer Mastitis verwendet wird, umfassend der Schritte:
(a) Bereitstellen einer Milchprobe der Milchkuh,
(b) Messen der Konzentration des Biomarkers Haptoglobin (HP) in der Milchprobe,
(c) Vergleich der gemessenen Konzentration aus (b) mit einem Referenzwert des gemessenen Biomarkers, wobei eine Abweichung der gemessenen Konzentration von dem Referenzwert einen nicht gesunden Zustand der Milchkuh anzeigt,
und wobei der Gesundheitszustand leichte systemische Erkrankung, leichte systemische Erkrankung mit Labmagenverlagerung (LMV), schwere systemische Erkrankung, oder Kombinationen dieser Erkrankungen umfasst.

In einem zweiten Aspekt wird die gestellte Aufgabe durch ein nicht-invasives Verfahren zur Überwachung des Gesundheitszustandes einer Milchkuh gelöst, umfassend der Schritte:
(a) Bereitstellen einer Milchprobe der Milchkuh,
(b) Messen der Konzentration der Biomarker Haptoglobin (HP) und sekretorischen Komponente des polymeren Immunglobulinrezeptors (PIGR (SC)) in der Milchprobe,
(c) Vergleich der gemessenen Konzentration aus (b) mit einem Referenzwert des zumindest einen gemessenen Biomarkers, wobei eine Abweichung der gemessenen Konzentration von dem Referenzwert einen nicht gesunden Zustand der Milchkuh anzeigt.

Vorzugsweise wird das nicht-invasive Verfahren vollständig *ex vivo* oder *in vitro* durchgeführt. Dabei ist insbesondere herauszustellen, dass die Biomarker der vorliegenden Erfindung in einer Milchprobe untersucht werden, sodass die Durchführung des Verfahrens unabhängig von einer invasiven Probeentnahme und damit unabhängig von einem Tierarzt ist. Dies ermöglicht es dem vorliegenden Verfahren auf große Milchkuhbestände und auf regelmäßige (monatliche) Testungen ausgeweitet zur werden, was beispielsweise durch die Überprüfung von Biomarkern in einer Blutprobe nicht wirtschaftlich durchführbar ist.

Die Begriffe "Protein-Biomarker", "Biomarker" und "Marker" werden für die vorliegende Beschreibung synonym verwendet. Die Begriffe bezeichnen bevorzugt die Konzentration einzelner, oder Kombinationen, biologischer Moleküle, wie Proteine, Nukleinsäuren, Kohlenhydrate usw. Insbesondere beschäftigt sich die vorliegende Offenbarung mit Proteinen als Biomarker. Soweit sich die Offenbarung auf eine Messung der Konzentration der Biomarker bezieht, soll sowohl eine direkte Messung der Konzentration (der Anzahl der Proteinmoleküle/Volumen oder Gewicht) als auch die indirekte Messung umfasst sein. So können auch Abbauprodukte der Proteinmarker der Erfindung gemessen werden, oder alternativ auf Basis der biochemischen Charakteristika der Biomarker auf deren Konzentration zurückgeschlossen werden. Enzyme können beispielweise über Nachweis ihrer enzymatischen Aktivität bestimmt werden.

Soweit erforderlich kann das Verfahren der vorliegenden Erfindung optional in Schritt (b) das Messen zumindest eines weiteren Biomarkers umfassen. Der zumindest eine weitere Biomarker wird hierzu vorzugsweise aus der Gruppe bestehend aus S100 Kalzium-bindendem Protein A9 (S 100A9), Interleukin (IL-) 18, Tumornekrosefaktor (TNF)-alpha, Laktoferrin (LTF), und *Vascular Endothelial Growth Factor* (VEGF) ausgesucht.

Eine weitere optionale und bevorzugte Weiterbildung der vorliegenden Erfindung ist ein Verfahren, worin Schritt (b) das Messen einer Kombination von mindestens drei Biomarker umfasst, nämlich HP, PIGR (SC) und einen dritten Biomarker ausgesucht aus der Gruppe bestehend aus S100A9, IL-18, TNF-alpha, LTF, und VEGF. Durch die Messung weiterer Biomarker kann die Spezifität und Sensitivität des Verfahrens weiter verbessert werden.

Bevorzugt ist ein hier beschriebenes Verfahren, wobei eine Abweichung der gemessenen Konzentration der Biomarker HP und PIGR (SC) von dem Referenzwert eine systemische Erkrankung der Milchkuh anzeigt, vorzugsweise eine systemische Erkrankung die nicht oder nicht-ausschließlich am Euter auftritt, beispielsweise leichte systemische Erkrankung, leichte systemische Erkrankung mit LMV, schwere systemische Erkrankung, oder Kombinationen dieser Erkrankungen. Vorzugsweise handelt es sich bei der gemessenen Abweichung um eine Erhöhung der Konzentration der Biomarker in der Probe einer erkrankten Kuh im Vergleich zu einer gesunden Kuh.

Die vorliegende Erfindung kann anhand der offenbarten Biomarker in einem Aspekt eine systemische Erkrankung, vorzugsweise außerhalb des Euters diagnostiziert werden. Alternativ betrifft die Erfindung jedoch auch die Diagnose einer Mastitis anhand der offenbarten Biomarker. Für diesen Aspekt ist es in einer Ausführungsform der Erfindung bevorzugt, dass eine Diagnose der Mastitis bei einer Milchkuh mittels Bestimmung einer Kombination der Biomarker HP und PIGR (SC) durchgeführt wird.

Der Begriff "Referenzwert" soll hier weit ausgelegt werden und eine Vielzahl möglicher Vergleichswerte umfassen. Je nach diagnostischem Ziel wird ein angemessener Referenzwert ausgesucht. Zu Erkennung erkrankter Tiere kann es sich bei dem Referenzwert beispielsweise um einen Wert des Biomarkers in einer gesunden Kuh handeln. Wird das Verfahren jedoch zur Überwachung eines Krankheitsverlaufs oder zur Überwachung einer Therapie einer erkrankten Kuh eingesetzt, kann der Referenzwert auch eine Konzentration des Biomarkers in der Milch der überwachten Kuh zu einem früheren Zeitpunkt - beispielsweise vor Beginn der Therapie - darstellen. Insbesondere ist jedoch bevorzugt, dass der Referenzwert ein Grenzwert (Cut-Off) ist und wobei, wenn die gemessene Konzentration des Biomarkers den Grenzwert überschreitet, ein nicht gesunder Zustand der Milchkuh festgestellt wird. Je nachdem wie der Cut-Off Wert gewählt ist, kann ein nicht gesunder Zustand bei einem Testwert größer als, oder größer/gleich als der Cut-off festgestellt werden. Ein Grenzwert kann zudem herdenspezifisch ermittelt werden, wobei eine zu überwachende gesunde Milchkuhherde regelmäßig auf den Biomarker getestet wird und anhand dieser Werte ein "gesunder" Referenzwert spezifisch für die Herde ermittelt wird. Fällt nun bei der regelmäßigen Überwachung der Herde eine Kuh durch eine erhöhte Konzentration des Biomarkers auf, ist ein nicht gesunder Zustand der Kuh anzunehmen.

In bevorzugten Ausführungsformen der vorliegenden Erfindung können die Grenzwerte (Cut-Off) zur Unterscheidung gesunder von kranken Tieren so gewählt werden, dass der entsprechende Biomarker zumindest 90 % Spezifität oder höher, vorzugsweise 92 %, weiter vorzugsweise 94 % oder höher aufweist, bei einer Sensitivität von zumindest 50 %, vorzugsweise mindestens 60 %, 70 % oder zumindest 80 %. So kann der Grenzwert für den Marker HP beispielsweise ungefähr 0,4 µg/ml betragen, vorzugsweise ungefähr 0,5 µg/ml und meist bevorzugt ungefähr 0,58 µg/ml. Der Grenzwert für den Marker PIGR (SC) kann beispielsweise ungefähr 5 µg/ml betragen, vorzugsweise ungefähr 8 µg/ml und meist bevorzugt ungefähr 8,2 µg/ml. Der Grenzwert für den Marker LTF kann beispielsweise ungefähr 80 µg/ml betragen, vorzugsweise ungefähr 100 µg/ml und meist bevorzugt ungefähr 120 µg/ml. Der Grenzwert für den Marker VEGF kann beispielsweise ungefähr 7 µg/ml betragen, vorzugsweise ungefähr 9 µg/ml und meist bevorzugt ungefähr 9,5 µg/ml. Der Begriff "ungefähr" in Zusammenhang mit numerischen Angaben lässt bevorzugter Weise eine Abweichung +/- 20 % des angegebenen Wertes zu, weiter bevorzugt eine Abweichung von +/- 15 %, +/-10 %, und meist bevorzugt +/-5 %.

Wie oben erwähnt eignet sich das erfinderische nicht-invasive Verfahren zur Überwachung einer Therapie einer Milchkuh, wobei eine Verringerung der Konzentration des Biomarkers während oder nach einer Therapie einen Therapieerfolg anzeigt. Da die Erhöhung der hier beschriebenen Biomarker in der Milch mit einer Verschlechterung des Gesundheitszustands einer Milchkuh korreliert, kann das Verfahren auch angewendet werden um den Therapieerfolg einzelner erkrankter Tiere zu überwachen. Dabei ist der Referenzwert, mit dem eine gemessene Konzentration des oder der Biomarker der Erfindung in der Milchprobe verglichen wird, beispielsweise eine Konzentration des oder der Biomarker in der Milch derselben Kuh an einem früheren Zeitpunkt, beispielsweise zu Beginn der Therapie.

In einigen Ausführungsformen ist es bevorzugt, dass die Konzentration des Biomarkers HP in einer unverdünnten Milchprobe bestimmt wird. In anderen Ausführungsformen ist die Milchprobe eine mit Konservierungsstoffen versetzte Milchprobe (z.B. bei der Milchleistungsprüfung).

Das Verfahren der vorliegenden Erfindung kann insbesondere routinemäßig zur Überwachung einer Milchkuh, oder Milchkuhherde eingesetzt werden. Daher ist es bevorzugt, dass das nicht-invasive Verfahren regelmäßig ausgeführt wird, vorzugsweise monatlich, weiter vorzugsweise wöchentlich, noch weiter vorzugsweise mehrmals pro Woche, bis täglich.

Die zu untersuchende Milchprobe der Milchkuh wird bei der Durchführung des erfindungsgemäßen Verfahrens vorzugsweise während eines Melkvorgangs aus der gemolkenen Milch (nicht-invasiv) entnommen. Bei vollautomatisierten Melkvorrichtungen kann die Milchprobe einer Milchkuh direkt aus der gemolkenen Milch (automatisiert) abgezweigt werden. Die so bereitgestellte Milchprobe wird dann für das hier beschriebene erfindungsgemäße Verfahren eingesetzt.

Zur Messung der Konzentration der Biomarker der vorliegenden Erfindung kann auf verschiedene dem Fachmann bekannte technische Verfahren zurückgegriffen werden. Insbesondere soll die vorliegende Offenbarung als nicht auf einzelne Analyseverfahren beschränkt angesehen werden. Die Bestimmung der Konzentration von Biomarkern in einer Milchprobe kann das Messen der Konzentration biochemisch mittels eines Verfahrens ausgesucht aus Sodium-Dodecyl-Sulfat-Polyacrylamid-Gelelektrophorese (SDS-PAGE), *Fast Protein Liquid Chromatography* (FPLC), und *High Performance Liquid Chromatography* (HPLC) umfassen, oder das Messen der Konzentration immunologisch mittels eines Verfahrens ausgesucht aus *Enzyme-linked Immunosorbent Assay* (ELISA), Enzym-Immuno-Assay (EIA), Fluoreszenz-Immuno-Assay (FIA), Chemilumineszenz-Immuno-Assay (CIA), Radio-Immuno-Assay (RIA), Western-Blot, und Peptid-Arrays umfassen, oder das Messen der Konzentration spektrometrisch mittels eines Verfahrens ausgesucht aus *Surface*-Plasmon-Resonanz (SPR), *Matrix-assisted Laser Desorption* / *Ionization* (MALDI) oder *Electrospray Ionization* (ESI) umfassen. Besonders bevorzugt sind immunologische Verfahren, beispielsweise mittels monoklonaler (bevorzugt) oder polyklonaler Antikörper, beispielsweise in einem ELISA.

Das beschriebene Verfahren soll insbesondere zur Überwachung des Gesundheitszustands einer oder mehrerer Milchkühe verwendet werden. Daher ist es in einigen Ausführungsformen beabsichtigt, dass das Verfahren zur Feststellung eines nicht gesunden Zustands einer Milchkuh verwendet wird. Eine konkretisierte Diagnose der vorliegenden Erkrankung der als nicht gesund klassifizierten Milchkuh ist in dieser Ausführungsform nicht zwingend notwendig. Vielmehr soll mittels Durchführung weiterer anschließender Diagnoseverfahren ermittelt werden, an welcher Erkrankung die identifizierte nicht-gesunde Milchkuh leidet. In diesen Ausführungsformen richtet sich das Verfahren der vorliegenden Offenbarung auf die möglichst frühe Erkennung auffälliger Tiere durch regelmäßige Untersuchungen gemäß der vorliegenden Erfindung. Im Falle eines festgestellten nicht gesunden Zustands kann im Nachhinein ein Tierarzt zur Etablierung einer spezifischen Diagnose hinzugezogen werden.

In einigen Ausführungsformen betrifft die vorliegende Offenbarung ein nicht-invasives Verfahren zur Überwachung des Gesundheitszustands einer Milchkuhherde in einem Milchbetrieb, umfassend ein regelmäßiges Durchführen eines Verfahrens zur Überwachung des Gesundheitszustandes mindestens einer, vorzugsweise jeder, Milchkuh der Milchkuhherde gemäß des hier beschriebenen nicht-invasiven Verfahrens zur Überwachung des Gesundheitszustandes einer Milchkuh.

Als Milchkuhherde soll ein Milchkuhbestand innerhalb eines Milchbetriebes von vorzugsweise mindestens zwei Tieren, weiter vorzugsweise von mindestens 5 Tieren, 10 Tieren, 15 Tieren, 20 Tieren, 50 Tieren oder mehr verstanden werden. Die vorliegende Erfindung ist besonders hilfreich zur Überwachung von Milchkuhherden mit mehr als 10 Tieren.

Die vorgenannte Aufgabe wird zudem in einem weiteren Aspekt durch ein nicht-invasives System und/oder Vorrichtung zur Überwachung des Gesundheitszustandes einer Milchkuh, umfassend:
(a) Mittel zur Aufnahme einer Milchprobe der Milchkuh,
(b) Antikörper zur immunologischen Messung der Konzentration der Biomarker HP und PIGR (SC) in der Milchprobe,
(c) Mittel zum Vergleich der gemessenen Konzentration aus (b) mit einem Referenzwert des gemessenen Biomarkers, wobei eine Abweichung der gemessenen Konzentration von dem Referenzwert einen nicht gesunden Zustand der Milchkuh anzeigt,
gelöst.

In einigen Ausführungsformen kann das nicht-invasive System und/oder Vorrichtung weiter Mittel zur Speicherung von Daten und/oder Mittel zur optischen Anzeige von Daten, wie beispielsweise ein Bildschirm, umfassen.

In einigen Ausführungsformen umfasst das nicht-invasive System und/oder Vorrichtung Mittel zur Ausgabe einer Information. Die Mittel zur Ausgabe einer Information sind dabei geeignet im Falle einer Feststellung eines nicht-gesunden Zustands einer Milchkuh, durch geeignete Mittel auszugeben. Beispielsweise kann bei Feststellung eines nicht gesunden Zustands einer Milchkuh ein optischer oder akustischer Alarm ausgelöst werden. Vorzugsweise wird zumindest eine Information über die Identität der identifizierten nicht-gesunden Milchkuh bereitgestellt.

Vorzugsweise ist das hier beschriebene System und/oder Vorrichtung an eine Melkanlage (Melksystem) angeschlossen, und umfasst Mittel, die während eines Melkvorgangs eine Milchprobe der Milchkuh von der Melkanlage an die Mittel zur Aufnahme einer Milchprobe abgeben. Automatisierte Melkanlagen, die Milchprobeentnahmen ermöglichen sind dem Fachmann bekannt.

Das nicht-invasive System und/oder Vorrichtung gemäß der vorliegenden Erfindung umfasst bevorzugt weiter Antikörper zur Messung der Konzentration zumindest eines weiteren Biomarkers ausgesucht aus der Gruppe bestehend aus S100A9, IL-18, TNF-alpha, LTF, und VEGF.

Das nicht-invasive System und/oder Vorrichtung gemäß der vorliegenden Erfindung ist daher geeignet zur Durchführung eines hier beschrieben Verfahrens.

Die Aufgabe der Erfindung wird weiter durch ein diagnostisches Kit zur Überwachung des Gesundheitszustands einer Milchkuh gelöst, umfassend Antikörper zur immunologischen Bestimmung oder Messung der Konzentration der Biomarker HP und PIGR (SC) in einer Milchprobe.

Das diagnostische Kit gemäß der Erfindung umfasst in einigen Ausführungsformen Mittel zur Durchführung eines Verfahrens ausgesucht aus EIA, FIA, CIA, RIA, Western-Blot, und Peptid-Arrays. Insbesondere ist das diagnostische Kit geeignet zur Durchführung eines hier beschriebenen Verfahrens zur Überwachung des Gesundheitszustands von Milchkühen.

Die vorliegende Erfindung wird nun mittels nicht einschränkender Beispiele weiter beschrieben.

In den Abbildungen wird gezeigt:
- **Abbildung 1:**: mRNA-Expression ausgesuchter Marker in Milchzellen (MZ) und Blutleukozyten (BL) von Kühen in unterschiedlichen Krankheitszuständen. Die Konzentration der Marker wurde mittels qPCR ermittelt und ist als Prozent der Expression der Referenzgene Cyclophilin B (PPIB) und ubiquitär exprimiertes Transkript (UXT) angegeben. system.: systemisch; Erkrank.: Erkrankung; MZ: Milchzellen; BL: Blutleukozyten; * 0,05 > p > 0,01, und ** p ≤ 0,01.
- **Abbildung 2:**: Konzentrationen potenzieller Protein-Biomarker in Milch. Die Konzentrationen wurden mittels kommerziell erhältlicher ELISA-Kits bestimmt. n.d. = nicht detektierbar; system.: systemisch; Erkrank.: Erkrankung; * 0,05 > p > 0,01, und ** p ≤ 0,01.
- **Abbildung 3:**: Korrelationen der Konzentrationen der Biomarker HP und LTF in Milch und Plasma. Die Konzentrationen wurden mittels kommerziell erhältlicher ELISA-Kits bestimmt. Positive Korrelationen werden durch die Regressionsgeraden angezeigt.
- **Abbildung 4:**: ROC-Kurven von ausgesuchten Milch-Biomarkern. A: ROC-Analyse der einzelnen Marker bei verschiedenen Krankheitszuständen. B: Zusammengefasste ROC-Analyse aller erkrankten Tiere. system.: systemisch; Erkrank.: Erkrankung

### BEISPIELE

### Material und Methoden:

### Quantifizierung von Protein-Biomarkern in Milch und Plasma

Ausgewählte Proteine in Milch und Plasma wurden mit im Handel erhältlichen ELISA-Kits quantifiziert. Alle HP-Messungen wurden in unverdünnten Proben durchgeführt, da dies ausreicht, um Schwankungen des Markers HP in verschiedenen Stadien der Erkrankung zu erkennen. Vorbeschichtete Platten wurden mit 100 µl Probe (30 min, Raumtemperatur (RT)) inkubiert. Gereinigtes HP (LeeBioSolutions, St. Louis, Missouri, USA) wurde als Standard in einem Bereich von 8 bis 0,125 µg/ml eingesetzt. Die Platte wurde 3-mal in Assay-Waschpuffer gewaschen und dann mit 100 µl 1:40 verdünnten Peroxidase-konjugierten Anti-HP-Antikörper (30 min, RT) inkubiert. Nach 3 Waschschritten wurden 100 µL gebrauchsfertige Tetramethylbenzidinsubstratlösung (Moss Inc., Pasadena, Maryland, USA) hinzugefügt, und 10 bis 30 Minuten bei RT inkubiert. Die Reaktion wurde mit 50 µl 9,9 % H₃PO₄ gestoppt.

PIGR (SC) wurde mit einem ELISA-Kit zum Nachweis von bovinem PIGR (Life Science USCN Inc.) nach Angaben des Herstellers quantifiziert. Milch wurde jeweils 1:300 bis 1:1.000 für die Kontrollproben und 1:5.000 bis 1:10.0000 für Proben von erkrankten Kühen verdünnt. Plasmaproben wurden 1:100.000 verdünnt.

### Statistische Analyse

Die Analyse der Unterschiede zwischen den Gruppen, Spearman-Rangkorrelationen, *Receiver-Operating-Characteristic* (ROC)-Analyse und Visualisierung der Ergebnisse wurden mittels SigmaPlot11 Software (Systat Software, Erkrath, Deutschland) durchgeführt. Um ungewollte statistische Neigungen zu vermeiden, wurden Proben der Tiere nach dem Zufallsprinzip für die Analyse durch quantitative *real-time-*RT-PCR (qPCR) oder ELISA ausgewählt. Datensätze wurden auf Normalverteilung analysiert. Wenn der Shapiro-Wilk-Test positiv absolviert wurde, wurde ein t-Test durchgeführt. Falls Daten nicht normal verteilt waren, wurde der Mann-Whitney-Rangsummentest verwendet. Alle erkrankten Gruppen wurden mit der Kontrollgruppe verglichen. Die Daten verschiedener Erkrankungen außerhalb des Euters wurden kombiniert, wenn eine geringe Anzahl von Proben getestet wurde. P-Werte sind folgendermaßen definiert: * 0,05 > p > 0,01, und ** p ≤ 0,01.

### Auswahl und Evaluation potenzieller Biomarker

Die ROC-Analyse wurde verwendet, um das Abgrenzungsvermögen der Biomarker zu bewerten. Eine Fläche unter der Kurve (*Area Under the Curve,* AUC) > 0,9 wurde als hoch diskriminierend und AUC-Werte < 0,6 als nicht diskriminierend betrachtet. Biomarker wurden anhand der besten Unterscheidung zwischen leichter systemischer Erkrankung und der Kontrollgruppe ausgewählt. Statistische Auswertung von Biomarkern und Markerkombinationen wurde mit der *Open-Source Data Mining Software* TANAGRA durchgeführt. Um ein mögliches *Overfitting* zu verhindern wurde eine Kreuzvalidierung *(Cross Validation,* CV) durchgeführt (10-fach, 1 Wiederholung). Die Werte für die Sensitivität, Spezifität und Resubstitutionsfehlerrate wurden aus der CV übernommen. Die verschiedenen Erkrankungen wurden zu einer Gruppe zusammengefasst. Die Biomarker oder deren Kombinationen wurden auf der Grundlage ihrer Fähigkeit zur Abgrenzung erkrankter Kühe ausgewertet.

### Beispiel 1: Differentielle Genexpression von Biomarkern in Milch

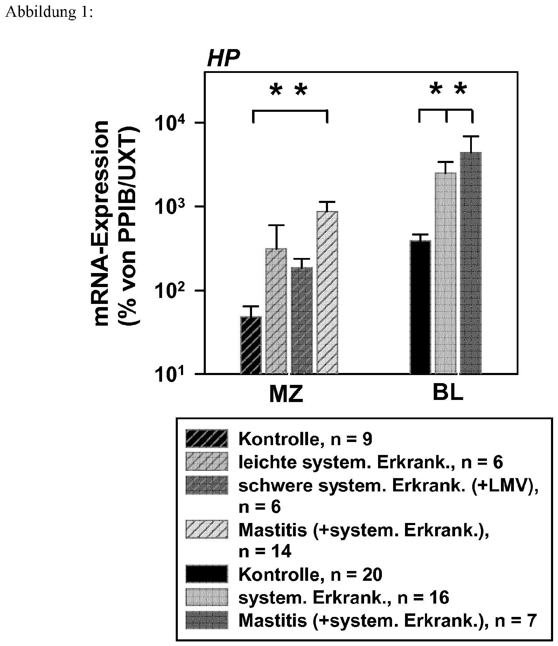

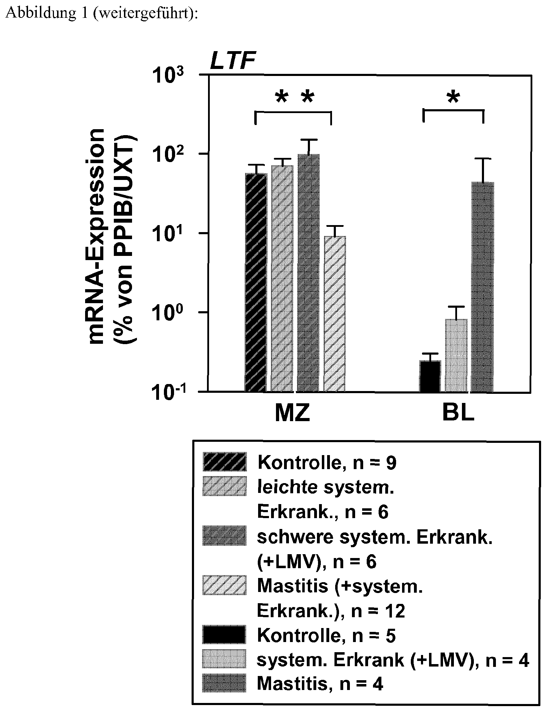

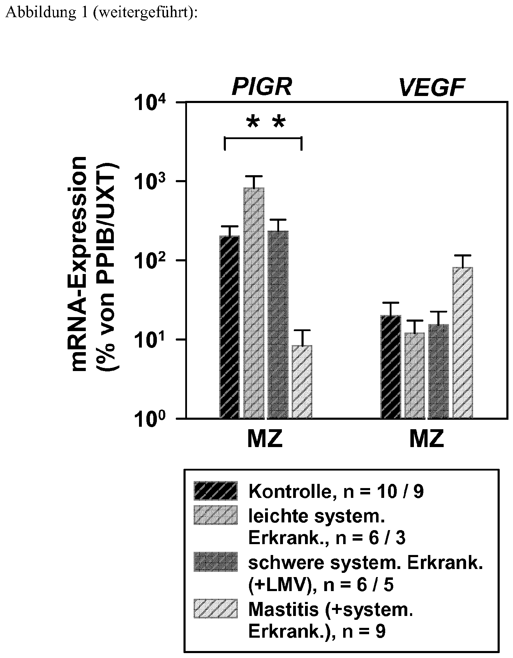

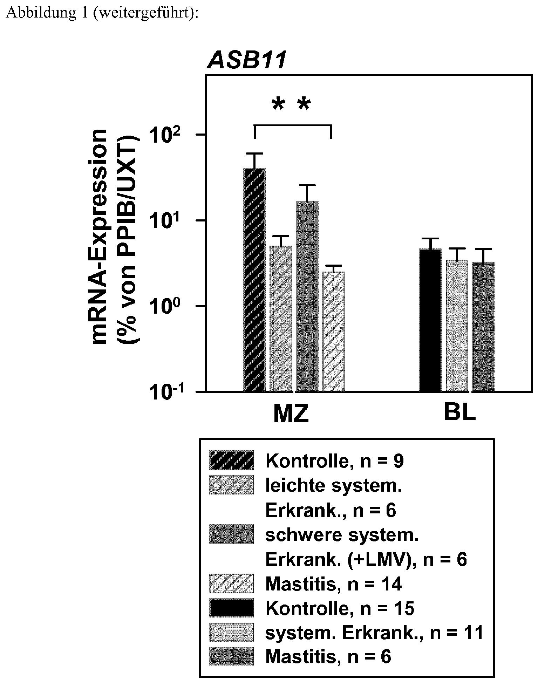

Die mRNA-Expression einzelner Biomarker in Milchzellen wurde mittels qPCR analysiert. Um die systemische Bedeutung möglicher Biomarker aus der örtlichen Umgebung der Brustdrüse zu bestätigen, wurde auch das Expressionsmuster der Biomarker in peripheren Blutleukozyten überprüft. Daten aus leichten und schweren systemischen Erkrankungsgruppen wurden im Falle einer geringen Probenzahl in einer systemischen Erkrankungsgruppe zusammengefasst und getestet. Abbildung 1 zeigt die Ergebnisse der relevantesten Biomarker.

### Beispiel 2: Quantifizierung und Auswahl der Biomarker

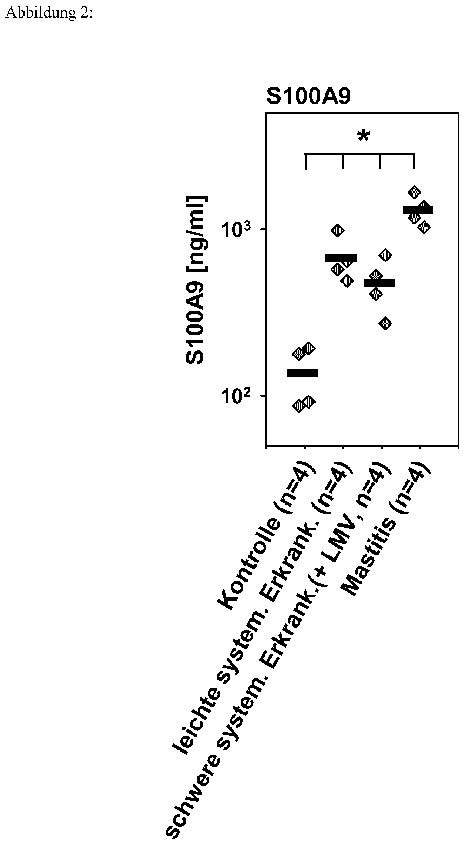

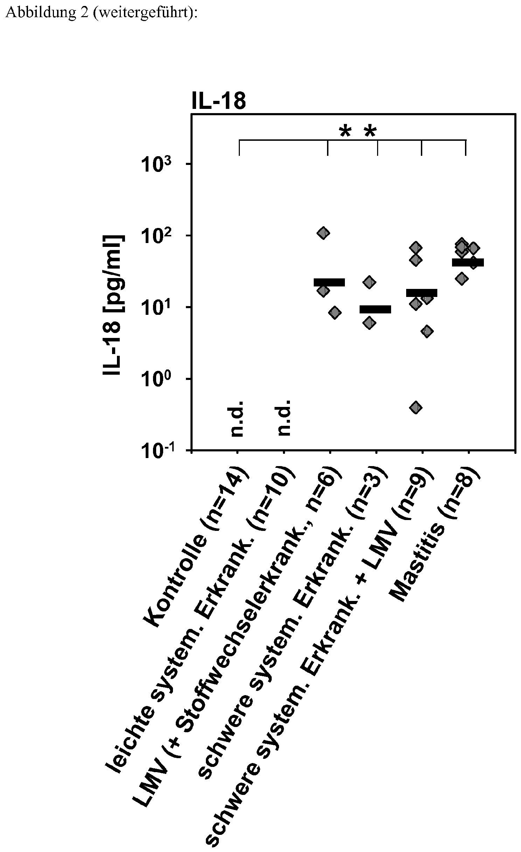

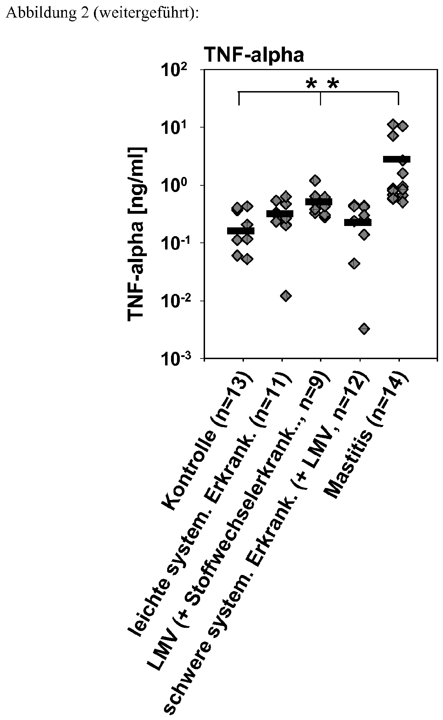

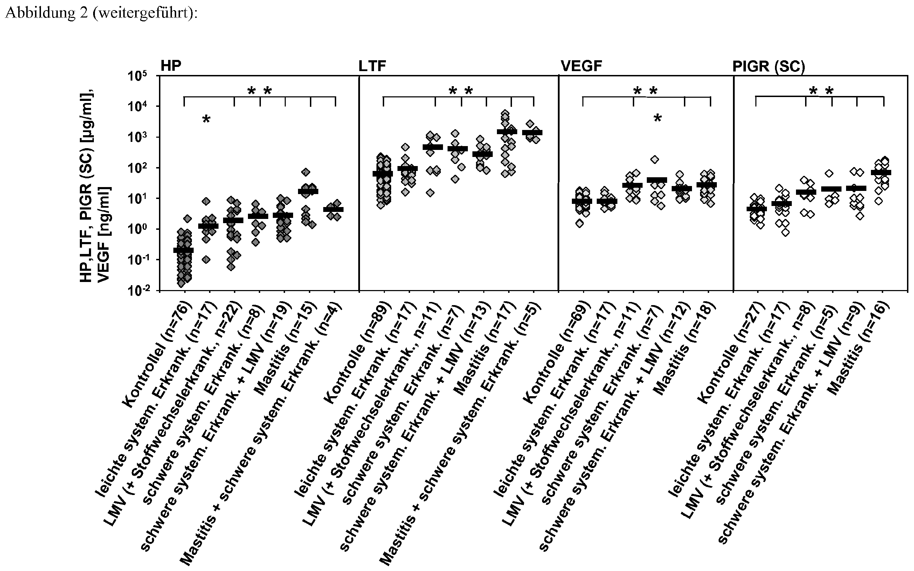

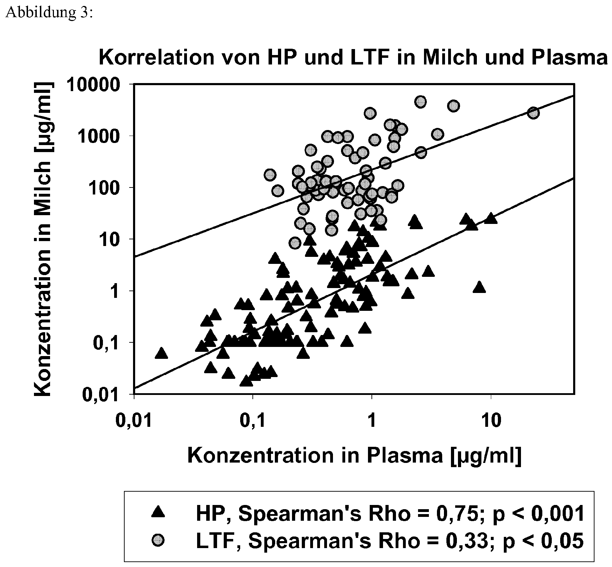

Auf Basis der Ergebnisse vorangegangener Experimente (Microarray, qPCR, und andere) wurden potenzielle Biomarker selektiert und auf Proteinebene mit kommerziellen ELISA-Kits quantifiziert. Es wurden erhöhte Konzentrationen von IL-18, LTF, PIGR (SC), TNF-alpha und VEGF in Milch bei LMV, schwerer systemischer Erkrankung, Mastitis und Kombinationen der Erkrankungen festgestellt. Allerdings zeigten HP und S100A9 erhöhte Werte bei leichter systemischer Erkrankung (Abbildung 2). Expressionsmuster von HP, IL-18 und LTF wurden auch im Plasma bestimmt, um die Aussagekraft der Marker für systemische Erkrankungen festzustellen. Die Korrelation von Milch- und Plasma-HP- und -LTF-Konzentrationen sind in Abbildung 3 gezeigt. Die positiven Spearman Korrelationskoeffizienten (Spearman ρ) zeigen den Zusammenhang von Milch- und Plasmaproteinkonzentrationen. Zusätzlich wurde die Korrelation der stärksten Biomarker in der Milch untersucht. Alle Proteine zeigten eine positive Korrelation der Konzentration in der Milch bei Erkrankungen (Tabelle 1). Die besten Marker wurden weiter statistisch evaluiert.

**Tabelle 1: Korrelationen von Protein-Biomarkern in Milch und Plasma**

| **Korrelation von** | **Spearman Korrelationskoeffizient** | **p** | **n** |
|---|---|---|---|
| **Korrelation in Milch** | | | |
| Milch HP und Milch PIGR (SC) | 0,67 | 0,001 | 71 |
| Milch LTF und Milch PIGR (SC) | 0,61 | 0,001 | 79 |
| Milch HP und Milch LTF | 0,59 | 0,001 | 142 |
| Milch HP und Milch VEGF | 0,58 | 0,001 | 120 |
| Milch LTF und Milch VEGF | 0,54 | 0,001 | 132 |
| Milch VEGF und Milch PIGR (SC) | 0,41 | 0,001 | 79 |

| **Korrelation in Milch und Plasma** | | | |
|---|---|---|---|
| Milch HP und Plasma HP | 0,78 | 0,001 | 121 |
| Milch IL-18 und Plasma IL-18 | 0,38 | 0,088 | 21 |
| Milch LTF und Plasma LTF | 0,33 | 0,005 | 69 |

| **Korrelation in Plasma** | | | |
|---|---|---|---|
| Plasma HP und Plasma LTF | 0,59 | 0,001 | 63 |

### Beispiel 3: Statistische Auswertung der Biomarker

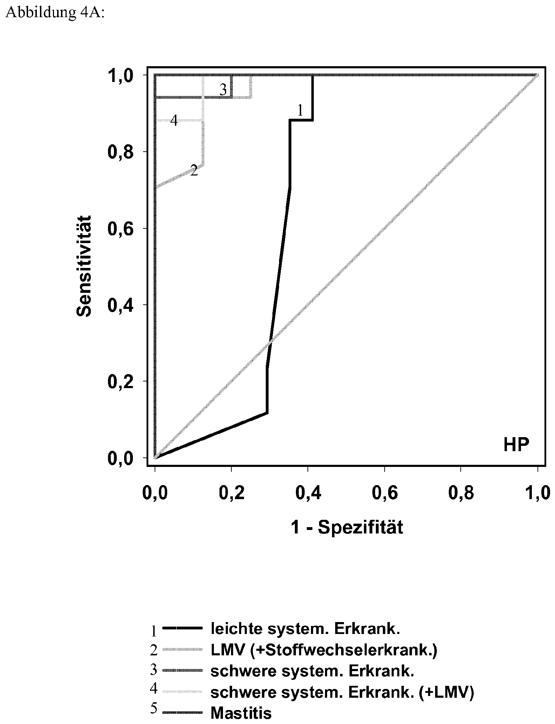

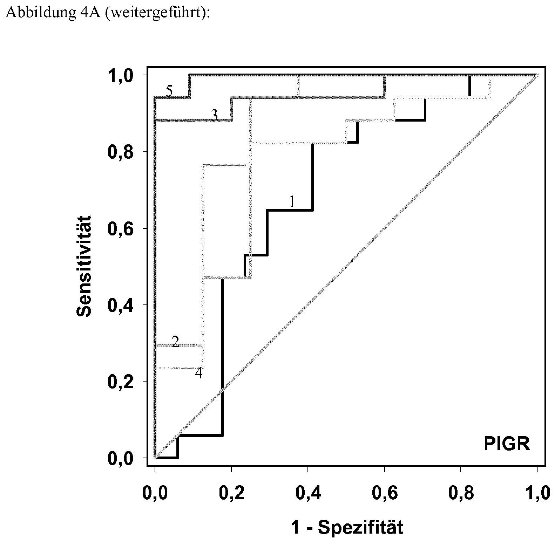

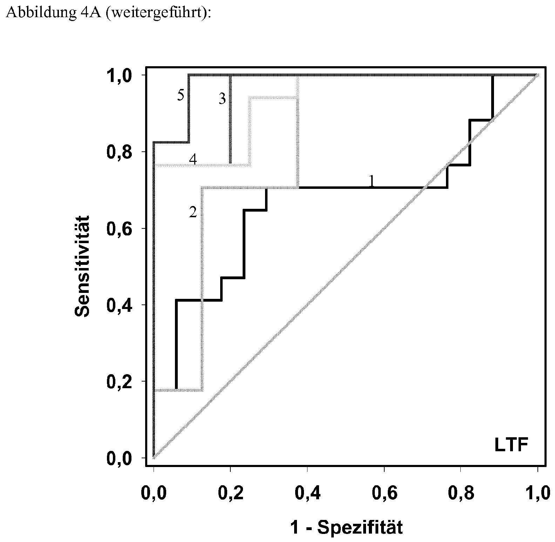

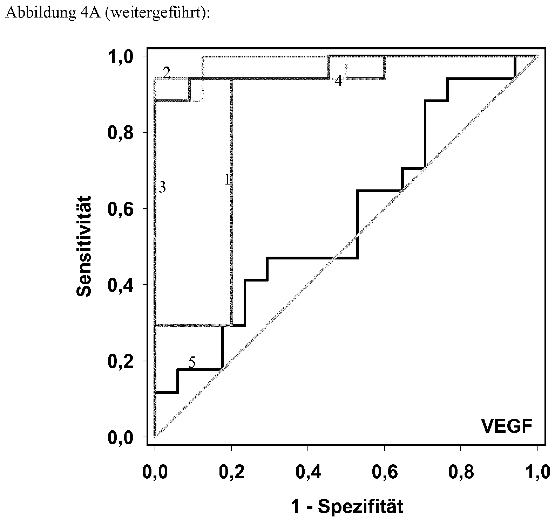

Zur statistischen Auswertung wurden die stark regulierten und hoch konzentrierten Milch-Biomarker HP, PIGR (SC), LTF und VEGF ausgewählt. Eine Untergruppe der Proben, bei denen alle vier Marker bestimmt worden waren, wurde verwendet, um einen direkten Vergleich der Ergebnisse zu ermöglichen. Die Biomarker allein und Kombinationen von zwei Biomarkern wurden bewertet. Dabei wurden 17 Kontrollproben und 49 Proben von erkrankten Kühen verwendet. Das Abgrenzungsvermögen für jede Erkrankungsgruppe wurde mittels ROC-Analyse (Abbildung 4A, Tabelle 2) bestimmt. HP und PIGR (SC) zeigten die beste Unterscheidung von leichter systemischer Erkrankung mit einer AUC von 0,69 und 0,68. Alle Proteine waren hoch diskriminierend für schwere systemische Erkrankungen und Mastitis (AUC > 0,9).

**Tabelle 2: Abgrenzungsvermögen von Milchbiomarkern für verschiedene Erkrankungen. Die Daten wurden mittels ROC-Analyse generiert. (Kontrolle: n = 17, Leichte systemische (system.) Erkrankung (Erkrank.): n = 17, LMV (+ Stoffwechselerkrank.): n = 8, Schwere system. Erkrank.: n = 5, Schwere system. Erkrank. + LMV: n = 8, Mastitis: n = 11)**

| **Kontrolle vs. Erkrankungsgruppe** | **AUC** | **95 % Konfidenzintervall** | **p** |
|---|---|---|---|
| **HP** | | | |
| Leichte system. Erkrank. | 0,69 | 0,48 - 0,89 | 0,065 |
| LMV (+ Stoffwechselerkrank.) | 0,96 | 0,89 - 1,03 | < 0,001 |
| Schwere system. Erkrank. | 0,99 | 0,95 - 1,03 | 0,001 |
| Schwere system. Erkrank. + LMV | 0,99 | 0,95 - 1,02 | < 0,001 |
| Mastitis | 1,00 | 1,00 - 1,00 | < 0,001 |

| **PIGR (SC)** | | | |
|---|---|---|---|
| Leichte system. Erkrank. | 0,68 | 0,49 - 0,87 | 0,071 |
| LMV (+Stoffwechselerkrank.) | 0,84 | 0,64 - 1,04 | < 0,05 |
| Schwere system. Erkrank. | 0,95 | 0,87 - 1,04 | < 0,05 |
| Schwere system. Erkrank. + LMV | 0,80 | 0,61 - 0,99 | < 0,05 |
| Mastitis | 0,99 | 0,98 - 1,01 | < 0,001 |

| **LTF** | | | |
|---|---|---|---|
| Leichte system. Erkrank. | 0,67 | 0,48 - 0,86 | 0,088 |
| LMV (+ Stoffwechselerkrank.) | 0,82 | 0,62 - 1,03 | < 0,05 |
| Schwere system. Erkrank. | 0,95 | 0,86 - 1,05 | < 0,05 |
| Schwere system. Erkrank. + LMV | 0,93 | 0,84 - 1,03 | < 0,001 |
| Mastitis | 0,98 | 0,95 - 1,02 | < 0,001 |

| **VEGF** | | | |
|---|---|---|---|
| Leichte system. Erkrank. | 0,57 | 0,38 - 0,77 | 0,459 |
| LMV (+Stoffwechselerkrank.) | 0,99 | 0,96 - 1,02 | < 0,001 |
| Schwere system. Erkrank. | 0,84 | 0,58 - 1,08 | < 0,05 |
| Schwere system. Erkrank. + LMV | 0,96 | 0,90 - 1,03 | < 0,001 |
| Mastitis | 0,97 | 0,91 - 1,03 | < 0,001 |

Markerkombinationen wurden zur Diskriminierung zwischen kranken und Kontrolltieren mit zwei statistischen Klassifikationsverfahren, der multinominalen logistischen Regression (MLR) und der k-nächste-Nachbarn-Klassifikation (K-NN) (Tabelle 4), ausgewertet. Ein zweites statistisches Modell wurde angewendet, um mögliche Verzerrungen der Ergebnisse zu vermeiden. HP ist die beste Wahl für den Einsatz als Einzelbiomarker. In Kombination mit PIGR (SC) oder LTF kann eine leichte Steigerung der Sensitivität oder Spezifität erzielt werden. Diese Kombinationen zeigten die besten Ergebnisse zur Detektion von erkrankten Tieren.

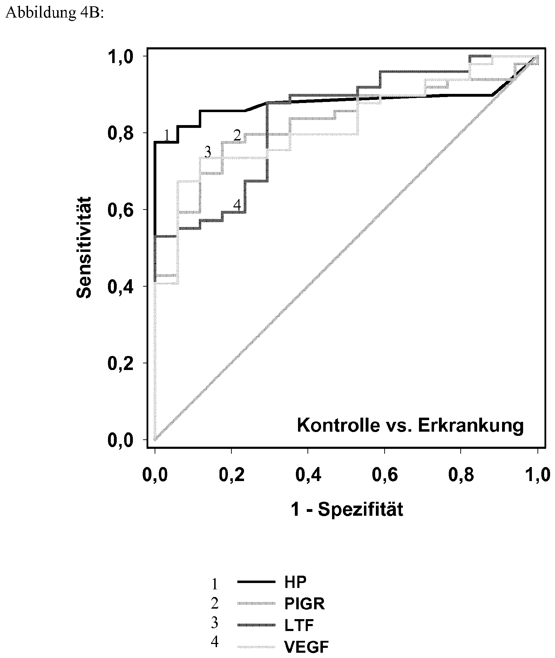

Eine praktische Anwendung von Biomarkern verlangt hohe Spezifität des Tests, um das Auftreten von Erkrankungen in großen Milchviehbetrieben nicht zu überschätzen. Daher wurde eine ROC-Analyse kombiniert für alle Krankheitsgruppen vs. Kontrolle durchgeführt, um die Sensitivität ("richtig positiv"), Spezifität ("richtig negativ"), 1-Sensitivität ("falsch negativ") und 1-Spezifität ("falsch positiv") der Biomarker-Bestimmung in Milch bei unterschiedlichen Grenzwert (Cut-Off)-Konzentrationen zu bewerten. Tabelle 3 zeigt die Werte für mögliche Cut-Off-Konzentrationen bei einer hohen Spezifität von 94 %. Dementsprechende ROC-Kurven sind in Abbildung 4B gezeigt. Bei einer Spezifität von 94 %, würden 6 % der eigentlich gesunden Kühe als krank erkannt werden. 18 %, 41 %, 45 % und 33 % der kranken Tiere würden bei der Ermittlung von HP, PIGR (SC), LTF und VEGF jeweils als gesund diagnostiziert werden.

Basierend auf dieser Analyse konnte daher gezeigt werden, dass die Bestimmung von HP für den Nachweis von Erkrankungen bei Milchkühen geeignet ist. Eine kombinierte Messung mit PIGR (SC) oder LTF ist auch möglich, um die Sensitivität oder Spezifität zu erhöhen.

**Tabelle 3: Abgrenzungsvermögen von Milchbiomarkern für erkrankte Tiere. Die Daten wurden mittels ROC-Analyse generiert. (Kontrolle: n = 17, erkrankt: n = 49)**

| **AUC** | **95 % Konfidenzintervall** | **p** | **Cut-Off bei 94 % Spezifität** | **Sensitivität bei 94 % Spezifität / %** |
|---|---|---|---|---|
| **HP** | | | | |
| 0,88 | 0,80 - 0,96 | < 0,001 | 0,58 µg/ml | 82 |

| **PIGR (SC)** | | | | |
|---|---|---|---|---|
| 0,82 | 0,72 - 0,93 | < 0,001 | 8,20 µg/ml | 59 |

| **LTF** | | | | |
|---|---|---|---|---|
| 0,84 | 0,74 - 0,94 | < 0,001 | 120,7 µg/ml | 55 |

| **VEGF** | | | | |
|---|---|---|---|---|
| 0,82 | 0,72 - 0,92 | < 0,001 | 9,50 ng/ml | 67 |

**Tabelle 4: Evaluation von Milchbiomarkern und deren Kombinationen. Die Klassifikation wurde mittels MLR und K-NN durchgeführt: Kontrolle (n = 17) vs. erkrankt (n = 49). Sensitivität, Spezifität und Resubstitutionsfehlerraten wurden aus der CV entnommen (10-fach, 1 Wiederholung).**

| | **Multinomiale logistische Regression k-nächste-Nachbarn Klassifikation** | | | | | |
|---|---|---|---|---|---|---|
| | **(Kreuzvalidierung) / %** | | | **(Kreuzvalidierung) / %** | | |
| **Marker (Kombination)** | **Sensitivität** | **Spezifität** | **Fehlerrate** | **Sensitivität** | **Spezifität** | **Fehlerrate** |
| **Einzelmarker** | | | | | | |
| **HP** | 86 | 88 | 13 | 91 | 69 | 15 |
| **LTF** | 84 | 44 | 27 | 82 | 63 | 23 |
| **VEGF** | 84 | 38 | 28 | 73 | 31 | 38 |
| **PIGR (SC)** | 86 | 25 | 30 | 77 | 19 | 38 |

| **Markerkombinationen** | | | | | | |
|---|---|---|---|---|---|---|
| **HP & VEGF** | 86 | 88 | 13 | 80 | 94 | 17 |
| **HP & PIGR (SC)** | 89 | 81 | 13 | 84 | 75 | 18 |
| **HP & LTF** | 89 | 69 | 17 | 86 | 81 | 15 |
| **VEGF & PIGR (SC)** | 86 | 63 | 20 | 82 | 56 | 25 |
| **LTF & PIGR (SC)** | 84 | 56 | 23 | 86 | 31 | 28 |
| **LTF& VEGF** | 82 | 56 | 25 | 84 | 44 | 27 |

### Abkürzungsverzeichnis

- AUC: *Area Under the Curve,* Fläche unter der Kurve
- BL: Blutleukozyten
- CIA: Chemilumineszenz-Immuno-Assay
- CV: *Cross Validation,* Kreuzvalidierung
- EIA: Enzym-Immuno-Assay
- ELISA: *Enzyme-linked Immunosorbent Assay*
- Erkrank.: Erkrankung
- ESI: *Electrospray Ionization*
- FIA: Fluoreszenz-Immuno-Assay
- FPLC: *Fast Protein Liquid Chromatography*
- HP: Haptoglobin
- HPLC: *High Performance Liquid Chromatography*
- Ig: Immunglobulin
- IL: Interleukin
- K-NN: k-nächste-Nachbarn-Klassifikation
- LMV: Labmagenverlagerung
- LTF: Laktoferrin
- MALDI: *Matrix-assisted Laser Desorption* / *Ionization*
- MLR: multinominale logistische Regression
- mRNA: *Messenger* Ribonukleinsäure
- MZ: Milchzellen
- PIGR: polymerer Immunglobulinrezeptor
- PPIB: Cyclophilin B (Referenzgen)
- RIA: Radio-Immuno-Assay
- ROC: *Receiver Operating Characteristic*
- S100A9: S100 Kalzium-bindendes Protein A9
- SC: *Secretory Component,* sekretorische Komponente des PIGR
- SDS-PAGE: *Sodium-*Dodecyl-Sulfat-Polyacrylamid-Gelelektrophorese
- SPR: *Surface*-Plasmon-Resonanz
- system.: systemisch
- TNF-alpha: Tumornekrosefaktor-alpha
- UXT: *Ubiquitously-Expressed Transcript,* ubiquitär exprimiertes Transkript (Referenzgen)
- VEGF: *Vascular Endothelial Growth Factor*

## Patentansprüche

1. Ein nicht-invasives Verfahren zur Überwachung des Gesundheitszustandes einer Milchkuh, wobei das Verfahren nicht zur Diagnose einer Mastitis verwendet wird, umfassend der Schritte:
(a) Bereitstellen einer Milchprobe der Milchkuh,
(b) Messen der Konzentration des Biomarkers Haptoglobin (HP) in der Milchprobe,
(c) Vergleich der gemessenen Konzentration aus (b) mit einem Referenzwert des gemessenen Biomarkers, wobei eine Abweichung der gemessenen Konzentration von dem Referenzwert einen nicht gesunden Zustand der Milchkuh anzeigt,
und wobei der Gesundheitszustand leichte systemische Erkrankung, leichte systemische Erkrankung mit Labmagenverlagerung (LMV), schwere systemische Erkrankung, oder Kombinationen dieser Erkrankungen umfasst.

2. Ein nicht-invasives Verfahren zur Überwachung des Gesundheitszustandes einer Milchkuh, umfassend der Schritte:
(a) Bereitstellen einer Milchprobe der Milchkuh,
(b) Messen der Konzentration der Biomarker Haptoglobin (HP) und sekretorischen Komponente des polymeren Immunglobulinrezeptor (PIGR (SC)) in der Milchprobe,
(c) Vergleich der gemessenen Konzentration aus (b) mit einem Referenzwert des zumindest einen gemessenen Biomarkers, wobei eine Abweichung der gemessenen Konzentration von dem Referenzwert einen nicht gesunden Zustand der Milchkuh anzeigt.

3. Das nicht-invasive Verfahren nach Anspruch 1 oder 2, wobei Schritt (b) das Messen zumindest eines weiteren Biomarkers ausgesucht aus der Gruppe bestehend aus LTF, S100A9, IL18, TNF-alpha, und VEGF, umfasst.

4. Das nicht-invasive Verfahren nach einem der Ansprüche 1 bis 3, wobei eine Abweichung der gemessenen Konzentration von dem Referenzwert eine systemische Erkrankung der Milchkuh anzeigt.

5. Das nicht-invasive Verfahren nach einem der Ansprüche 1 bis 4, wobei der Referenzwert ein Grenzwert (Cut-Off) ist, und wobei wenn die gemessene Konzentration des Biomarkers den Grenzwert überschreitet, ein nicht gesunder Zustand der Milchkuh festgestellt wird.

6. Das nicht-invasive Verfahren nach einem der Ansprüche 1 bis 5, wobei das Verfahren regelmäßig ausgeführt wird, vorzugsweise monatlich.

7. Das nicht-invasive Verfahren nach einem der Ansprüche 1 bis 6, wobei in Schritt (b) das Messen der Konzentration biochemisch mittels eines Verfahrens ausgesucht aus SDS-PAGE, FPLC und HPLC oder immunologisch mittels eines Verfahrens ausgesucht aus ELISA, EIA, FIA, CIA, RIA, Western-Blot und Peptid-Array, oder spektrometrisch mittels eines Verfahrens ausgesucht aus SPR, MALDI oder ESI, durchgeführt wird.

8. Ein nicht-invasives Verfahren zur Überwachung des Gesundheitszustands einer Milchkuhherde in einem Milchbetrieb, umfassend ein regelmäßiges Durchführen eines Verfahrens zur Überwachung des Gesundheitszustandes mindestens einer, vorzugsweise jeder, Milchkuh der Milchkuhherde gemäß eines der Ansprüche 1 bis 7.

9. Ein nicht-invasives System und/oder Vorrichtung zur Überwachung des Gesundheitszustandes einer Milchkuh, umfassend:
(a) Mittel zur Aufnahme einer Milchprobe der Milchkuh,
(b) Antikörper zur immunologischen Messung der Konzentration der Biomarker HP und PIGR (SC) in der Milchprobe,
(c) Mittel zum Vergleich der gemessenen Konzentration aus (b) mit einem Referenzwert des gemessenen Biomarkers, wobei eine Abweichung der gemessenen Konzentration von dem Referenzwert einen nicht gesunden Zustand der Milchkuh anzeigt.

10. Das nicht-invasive System und/oder Vorrichtung nach Anspruch 9, weiter umfassend Mittel zur Speicherung von Daten und/oder Mittel zur optischen Anzeige von Daten, wie beispielsweise ein Bildschirm.

11. Das nicht-invasive System und/oder Vorrichtung nach Anspruch 9 oder 10, wobei das System und/oder Vorrichtung an eine automatische oder halbautomatische Melkanlage (Melksystem) angeschlossen ist, und Mittel umfasst, die während eines Melkvorgangs eine Milchprobe der Milchkuh an die Mittel zur Aufnahme einer Milchprobe abgeben.

12. Ein diagnostisches Kit zur Überwachung des Gesundheitszustands einer Milchkuh, umfassend Antikörper zur immunologischen Bestimmung der Konzentration der Biomarker HP und PIGR (SC) in einer Milchprobe.

## Claims

1. A non-invasive method for monitoring the state of health of a dairy cow, the method not being used to diagnose mastitis, comprising the steps of:
(a) providing a milk sample from the dairy cow,
(b) measuring the concentration of the biomarker haptoglobin (HP) in the milk sample,
(c) comparing the measured concentration from (b) with a reference value for the measured biomarker, wherein a deviation of the measured concentration from the reference value indicates an unhealthy condition in the dairy cow,
and wherein the state of health includes minor systemic disease, minor systemic disease with abomasal displacement (LMV), serious systemic disease, or combinations of these diseases.

2. The non-invasive method for monitoring the state of health of a dairy cow, comprising the steps of:
(a) providing a milk sample from the dairy cow,
(b) measuring the concentration of the biomarkers haptoglobin (HP) and the secretory component of the polymeric immunoglobulin receptor (PIGR (SC)) in the milk sample,
(c) comparing the measured concentration from (b) with a reference value for the at least one measured biomarker, wherein a deviation of the measured concentration from the reference value indicates an unhealthy condition in the dairy cow.

3. The non-invasive method according to claim 1 or 2, wherein step (b) comprises the measurement of at least one further biomarker selected from the group consisting of LTF, S100A9, IL18, TNF-alpha, and VEGF.

4. The non-invasive method according to any one of claims 1 to 3, wherein a deviation of the measured concentration from the reference value indicates a systemic disease in the dairy cow.

5. The non-invasive method according to any one of claims 1 to 4, wherein the reference value is a threshold value (cut-off), and wherein if the measured concentration of the biomarker exceeds the threshold value, an unhealthy condition is determined to exist in the dairy cow.

6. The non-invasive method according to any one of claims 1 to 5, wherein the method is performed regularly, preferably monthly.

7. The non-invasive method according to any one of claims 1 to 6, wherein in step (b), the concentration is measured biochemically using a method selected from SDS-PAGE, FPLC and HPLC, measured immunologically using a method selected from ELISA, EIA, FIA, CIA, RIA, western blot and peptide array, or measured spectrometrically using a method selected SPR, MALDI or ESI.

8. The non-invasive method for monitoring the state of health of a dairy herd in a dairy farm, comprising regularly performing a method for monitoring the state of health of at least one, preferably each, dairy cow in the dairy herd according to any one of claims 1 to 7.

9. The non-invasive system and/or apparatus for monitoring the state of health of a dairy cow, comprising:
(a) means for collecting a milk sample from the dairy cow,
(b) antibodies for immunological measurement of the concentration of the biomarkers HP and PIGR (SC) in the milk sample,
(c) means for comparing the measured concentration from (b) with a reference value for the measured biomarker, wherein a deviation of the measured concentration from the reference value indicates an unhealthy condition in the dairy cow.

10. The non-invasive system and/or apparatus according to claim 9, further comprising means for storing data and/or means for visually displaying data, such as a screen.

11. The non-invasive system and/or apparatus according to claim 9 or 10, wherein the system and/or apparatus is connected to an automated or semi-automated milking plant (milking system) and comprises means that transfer a milk sample from the dairy cow during the milking process to the means for collecting a milk sample.

12. A diagnostic kit for monitoring the state of health of a dairy cow, comprising antibodies for immunologically determining the concentration of the biomarkers HP and PIGR (SC) in a milk sample.

## Revendications

1. Procédé non invasif permettant de surveiller l'état de santé d'une vache laitière, dans lequel le procédé n'est pas utilisé pour la diagnostique d'une mammite, comprenant les étapes consistant à :
(a) fournir un échantillon de lait de la vache laitière,
(b) mesurer la concentration du biomarqueur haptoglobine (HP) dans l'échantillon de lait,
(c) comparer la concentration mesurée en (b) avec une valeur de référence du biomarqueur mesuré, dans lequel un écart entre la concentration mesurée et la valeur de référence indique un état non sain de la vache laitière,
et dans lequel l'état de santé comprend une maladie systémique légère, une maladie systémique légère avec déplacement de caillette (LMV), une maladie systémique grave, ou des combinaisons de ces maladies.

2. Procédé non invasif permettant de surveiller l'état de santé d'une vache laitière, comprenant les étapes consistant à :
(a) fournir un échantillon de lait de la vache laitière,
(b) mesurer la concentration des biomarqueurs haptoglobine (HP) et composante sécrétoire du récepteur d'immunoglobuline polymère (PIGR (SC)) dans l'échantillon de lait,
(c) comparer la concentration mesurée en (b) avec une valeur de référence de l'au moins un biomarqueur mesuré, dans lequel un écart entre la concentration mesurée et la valeur de référence indique un état non sain de la vache laitière.

3. Procédé non invasif selon la revendication 1 ou 2, dans lequel l'étape (b) comprend la mesure d'au moins un autre biomarqueur choisi dans le groupe constitué par LTF, S100A9, IL18, TNF-alpha, et VEGF.

4. Procédé non invasif selon l'une des revendications 1 à 3, dans lequel un écart entre la concentration mesurée et la valeur de référence indique une maladie systémique de la vache laitière.

5. Procédé non invasif selon l'une des revendications 1 à 4, dans lequel la valeur de référence est une valeur limite (cut-off), et dans lequel, si la concentration mesurée du biomarqueur dépasse la valeur limite, un état non sain de la vache laitière est constaté.

6. Procédé non invasif selon l'une des revendications 1 à 5, dans lequel le procédé est exécuté régulièrement, de préférence mensuellement.

7. Procédé non invasif selon l'une des revendications 1 à 6, dans lequel, dans l'étape (b), la mesure de la concentration est effectuée par voie biochimique au moyen d'un procédé choisi parmi SDS-PAGE, FPLC et HPLC, ou par voie immunologique au moyen d'un procédé choisi parmi ELISA, EIA, FIA, CIA, RIA, Western-Blot et Peptid-Array, ou par voie spectrométrique au moyen d'un procédé choisi parmi SPR, MALDI ou ESI.

8. Procédé non invasif permettant de surveiller l'état de santé d'un troupeau de vaches laitières dans une exploitation laitière, comprenant la mise en œuvre régulière d'un procédé permettant de surveiller l'état de santé d'au moins une, de préférence de chaque, vache laitière du troupeau de vaches laitières selon l'une des revendications 1 à 7.

9. Système et/ou dispositif non invasif permettant de surveiller l'état de santé d'une vache laitière, comprenant :
(a) des moyens pour le prélèvement d'un échantillon de lait de la vache laitière,
(b) des anticorps pour la mesure immunologique de la concentration des biomarqueurs HP et PIGR (SC) dans l'échantillon de lait,
(c) des moyens pour la comparaison de la concentration mesurée en (b) avec une valeur de référence du biomarqueur mesuré, dans lequel un écart entre la concentration mesurée et la valeur de référence indique un état non sain de la vache laitière.

10. Système et/ou dispositif non invasif selon la revendication 9, comprenant en outre des moyens pour le stockage de données et/ou des moyens pour l'affichage visuel de données, comme par exemple un écran.

11. Système et/ou dispositif non invasif selon la revendication 9 ou 10, dans lequel le système et/ou dispositif est raccordé à une installation de traite automatique ou semi-automatique (système de traite), et comprend des moyens qui fournissent un échantillon de lait de la vache laitière aux moyens pour la réception d'un échantillon de lait pendant une opération de traite.

12. Kit de diagnostic permettant de surveiller l'état de santé d'une vache laitière, comprenant des anticorps pour la détermination immunologique de la concentration des biomarqueurs HP et PIGR (SC) dans un échantillon de lait.
